(19) Europäisches Patentamt
European Patent Office
Office européen des brevets

(11) Veröffentlichungsnummer: **0 000 042**
**B1**

(12) # EUROPÄISCHE PATENTSCHRIFT

(21) Anmeldenummer: **78100071.6**

(22) Anmeldetag: **01.06.78**

(51) Int. Cl.[3]: **C 07 D 239/30,** // C 07 C 155/08

(54) **Verfahren zur Herstellung von 2,4,5-Trichlorpyrimidin**

(30) Priorität: **08.06.77 DE 2725888**

(43) Veröffentlichungstag der Anmeldung:
**20.12.78 Patentblatt 78/01**

(45) Bekanntmachung des Hinweises auf die Patenterteilung:
**20.08.80 Patentblatt 80/17**

(84) Benannte Vertragsstaaten:
**CH DE FR GB**

(56) Entgegenhaltungen:
**FR - A - 1 545 314**

(73) Patentinhaber: **Bayer AG**
**Zentralbereich Patente, Marken und Lizenzen**
**D - 5090 Leverkusen 1, Bayerwerk (DE)**

(72) Erfinder: **Beck, Gunther, Dr.**
**Am Mittelberg 1**
**D - 5090 Leverkusen (DE)**
**Heitzer, Helmut, Dr.**
**Höhenstrasse 84**
**D - 5090 Leverkusen 3 (DE)**

**EP 0 000 042 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung von 2,4,5-Trichlorpyrimidin

Gegenstand der vorliegenden Erfindung ist ein neues Verfahren zur Herstellung von 2,4,5-Trichlorpyrimidin.

Das Verfahren ist dadurch gekennzeichnet, daß man (2-Cyanethyl)-dithiocarbamidsäureester der Formel

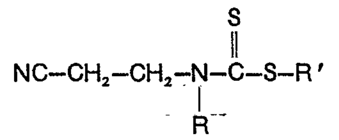

(I)

worin

R und R' einen $C_1$—$C_4$-Alkylrest bedeuten

gegebenenfalls in Mischung mit einem inerten Verdünnungsmittel bei Temperaturen von 0 bis 50°C, vorzugsweise 30—40°C, mit weniger als 7,5 Mol Chlor, insbesondere 3,5—7,0 Mol Chlor bezogen auf 1 Mol Ausgangsverbindung, umsetzt und anschließend in Abwesenheit von Chlor auf Temperaturen von 100—150°C, vorzugsweise 110—140°C erhitzt.

In einer bevorzugten Ausführungsform wird bei Temperaturen von etwa 20°C bis etwa 40°C chloriert, bis die exotherme Reaktion beendet ist und anschließend in Abwesenheit von Chlor auf Temperaturen von 110 bis 140°C nacherhitzt.

Gegebenenfalls kann man darüber hinaus nach dem Erhitzen auf 100—150°C nochmals bei 150—200°C, vorzugsweise 160—190°C, mit elementarem Chlor umsetzen. Dabei werden schwefelhaltige Nebenprodukte in 2,4,5-Trichlorpyrimidin umgewandelt, so daß sich in manchen Fällen die Ausbeute erhöhen läßt.

Das Chlor kann bei der Chlorierungsreaktion sowohl in Form von elementarem Chlor als auch in Form üblicher Chlorierungsmittel wie beispielsweise Schwefeldichlorid, Sulfurylchlorid, Phosphorpentachlorid eingesetzt werden. Dabei müssen selbstverständlich entsprechende Mengen Chlorierungsmittel verwendet werden, z.B. an Stelle von 1 Mol $Cl_2$ 2 Mol $SCl_2$.

Die Ausgangsverbindungen der Formel (I) sind zwar nicht bekannt, lassen sich jedoch leicht nach den Angaben der Literatur zur Darstellung von Dialkyldithiocarbamidsäurealkylestern (z.B. J. Chem. Soc. *1944,* S. 151) herstellen, indem man gemäß folgendem Formelschema

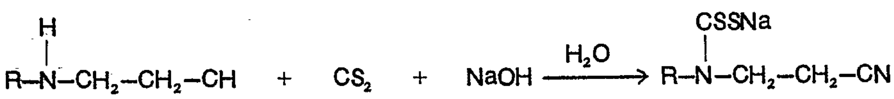


(II) (III)

zunächst cyanethylierte Amine (II), in denen R die oben genannte Bedeutung besitzt, mit Schwefelkohlenstoff in wäßriger Natronlauge zu den Dithiocarbamaten der Formel (III) umsetzt, welche anschließend durch Alkylierung in die Ester der Formel (I) übergeführt werden, z.B. mit (substituierten) Alkylhalogeniden, Sulfonsäurealkylestern oder Dialkylsulfaten, z.B. mit Dimethylsulfat gemäß folgendem Formelschema:

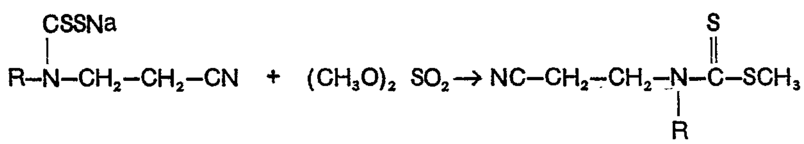

(III) (I) (R' = $CH_3$)

Die cyanethylierten Amine (II) erhält man beispielsweise nach folgendem Formelschema

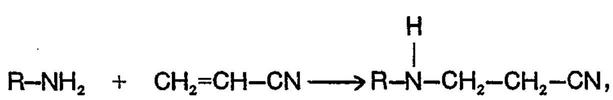

(IV) (II)

indem man primäre Amine (IV), in denen R die obengenannte Bedeutung besitzt, an Acrylnitril anlagert; (vgl. z.B. J. Am. Chem. Soc. *66*, 725 (1944);
J. Am. Chem. Soc. *68*, 1217 (1946);
J. Am. Chem. Soc. *78*, 2573 (1956);
J. Heterocyclic Chem. *1*, 260 (1964);

Für das erfindungsgemäße Verfahren geeignete (2 - Cyanethyl) - dithiocarbamidsäureester der Formel (I) sind z.B.:
(2-Cyanethyl)-methyl-dithiocarbamidsäuremethylester,
(2-Cyanethyl)-ethyl-dithiocarbamidsäuremethylester,
(2-Cyanethyl)-methyl-dithiocarbamidsäuremethylester,
(2-Cyanethyl)-ethyl-dithiocarbamidsäuremethylester,
(2-Cyanethyl)-methyl-dithiocarbamidsäurepropylester,
(2-Cyanethyl)-methyl-dithiocarbamidsäurebutylester,
(2-Cyanethyl)-butyl-dithiocarbamidsäuremethylester.

Verdünnungsmittel, die unter den Reaktionsbedingungen inert sind, sind alle gegen Chlor beständigen Lösungsmittel, z.B. chlorierte aliphatische und aromatische Kohlenwasserstoffe, wie Methylenchlorid, Chloroform, Tetrachlorkohlenstoff, 1,1,2,2-Tetrachloräthan, Tetrachlorethylen, 1,1,2,3,3-Pentachlorpropan, Hexachlorcyclopentadien, Oktachlorcyclopenten, 1,2,4-Trichlorbenzol, chlorierte Pyrimidine sowie Phosphoroxychlorid. Im allgemeinen werden pro Gew.- Teil (I) 0,5 bis 20, vorzugsweise 1 bis 10 Volumteile Verdünnungsmittel angewandt.

Für den Fall, daß das Chlorierungsmittel unter den Reaktionsbedindungen eine Flüssigkeit darstellt wie beispielsweise Schwefeldichlorid oder Sulfurylchlorid, kann die zusätzliche Verwendung eines inerten Verdünnungsmittels entfallen.

In Verbindung mit Schwefeldichlorid als Chlorierungsmittel kann besonders vorteilhaft Dischwefeldichlorid als Verdünnungsmittel verwendet werden.

Bei der Verwendung von Chlor als Chlorierungsmittel verläuft die Reaktion zu Beginn stark exotherm. Zweckmäßigerweise arbeitet man deshalb — insbesondere bei größeren Ansätzen — bis zum Abklingen der exothermen Reaktion nicht mit einem Überschuß an Chlor. Nach Abklingen der stark exothermen ersten Chlorierungsphase arbeitet man, um die Reaktion möglichst rasch zu beenden, zweckmäßigerweise mit einem Überschuß an Chlor (erkennbar an der grünlichen Farbe des Chlorierungsabgases).

Das Verfahren wird im einzelnen in der Weise durchgeführt, daß man zunächst ein (2-Cyanethyl-dithiocarbamidsäureester der Formel (I), insbesondere den (2-Cyanethyl)-methyl-dithiocarbamidsäuremethylester, mit einem der erwähnten Verdünnungsmittel, z.B. Chloroform, bei Raumtemperatur mischt und anschließend das Chlorierungsmittel zugibt. Außenkühlung und Dosierung des Chlorierungsmittels werden dabei so aufeinander abgestimmt, daß die anfangs stark exotherme Reaktion nicht zu heftig wird und 40°C nicht übersteigt.

Vorzugsweise wird so lange bei 30—40°C chloriert, bis die exotherme Reaktion völlig abgeklungen ist.

Anschließend zieht man im Vakuum unterhalb 40°C noch im Reaktionsgemisch gelöstes Chlor sowie das durch die Chlorierungsreaktion entstandene Schwefelchlorid ab und heizt den Rückstand entweder weiter in Vakuum oder bei Normaldruck unter Ausschluß von Feuchtigkeit bis auf etwa 120—130°C auf, wobei 2,4,5-Trichlorpyrimidin entsteht. Das 2,4,5-Trichlorpyrimidin wird destillativ, z.B. mit Hilfe einer Ag-verspiegelten Füllkörperkolonne von etwa 1 m Länge, abgetrennt. Es läßt sich leicht in einer Reinheit von über 99% gewinnen.

Arbeitet man mit einem unter den Reaktionsbedingungen flüssigen Chlorierungsmittel wie Schwefeldichlorid, so empfiehlt es sich, letzteres vorzulegen und das Ausgangsprodukt (I) bei 30—40°C anteilweise zuzudosieren.

Bei Verwendung von Schwefeldichlorid als Chlorierungsmittel ist es besonders vorteilhaft, weniger als 15 Mol $SCl_2$ pro Mol (I) (entsprechend den oben erwähnten weniger als 7,5 Mol $Cl_2$ pro Mol (I)) vorzulegen. Dadurch entfällt die Entfernung überschüssigen Chlorierungsmittels im Vakuum unterhalb 40°C.

2,4,5-Trichlorpyrimidin ist als Ausgangskomponenten für die Herstellung von Reaktivfarbstoffen geeignet (Umsetzung mit aminogruppenhaltigen Farbstoffen).

2,4,5-Trichlorpyrimidin kann durch Gasphasenchlorierung (GB-PS 1 201 228) in Tetrachlorpyrimidin umgewandelt werden. Tetrachlorpyrimidin ist als Reakativkomponente für die Herstellung von Reaktivfarbstoffen geeignet (vgl. z.B. Belgische Patentschrift Nr. 578 933). Darüber hinaus besitzt 2,4,5-Trichlorpyrimidin fungizide und sporizide Eigenschaften (vgl. US-Patentschrift Nr. 3 227 612).

In der Französischen Patentschrift 1 545 314 wird im Rahmen einer allgemeinen Formel die Herstellung von Trichlorpyrimidin durch Umsetzung von Aminopropinnitrilen der Formel

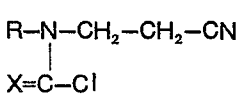

(X = 0 oder S)

(R = abspaltbarer Rest)

mit Chlor beschrieben. Ganz adgesehen davon, daß kein Beispiel für X=S angegeben ist, würde ein solches Verfahren gegenüber dem erfindungsgemäßen erhebliche Nachteile aufweisen, da die Ausgangsprodukte mit X=S nur schwierig herzustellen sind (Reaktion mit dem teuren und sehr giftigen Thiophosgen). Das beschriebene Verfahren legt das erfindungsgemäße in keiner Weise nahe.

Beispiel 1

In einem 2 1-Dreihalskolben, der mit Rührer, Tropftrichter und Thermometer versehen ist, werden 670 g (6,5 Mol) Schwefeldichlorid und (als Verdünnungsmittel) 250 ml Dischwefeldichlorid bei 35°C vorgelegt. Unter Rühren und gelegentilichem Kühlen mit Eiswasser werden nun im Verlauf etwa einer halben Stunde im Temperaturbereich von 35 bis 40°C 87 g (0,5 Mol) aufgeschmolzener (2-Cyanethyl)-methyl-dithiocarbamidsäuremethylester eingetropft. Nach beendeter Zugabe wird noch etwa eine viertel Stunde bis zur beendeten Gasentwicklung bei 35—40° nachgerührt. Anschließend wird die Reaktionsmischung unter Rühren in etwa 2 Stunden bis auf Rückfluß (137°C) erhitzt, wobei im Bereich an etwa 60°C ein dicker Niederschlag entsteht, der ab etwa 120°C wieder in Lösung geht. Die Bildung des 2,4,5-Trichlorpyrimidins ist beendet, wenn eine klare Lösung vorliegt und keine Gasentwicklung mehr beobachtet wird. Anschließend werden alle bis zu einer Badtemperatur von 150°C/14 Torr destillierbaren Anteile des Reaktionsgemisches abdestilliert. Das Destillat enthält nach gaschromatographischer Analyse 78 g (entsprechend 85% der Theorie) 2,4,5-Trichlorpyrimidin.

Leitet man in den Destillationsrückstand ab 150°C bis zu einer Innentemperatur von etwa 180°C 1/2—1 Stunde lang einen leicht überschüssigen Chlorstrom (erkennbar an der grünlichen Farbe des Abgases) und destilliert anschließend im Wasserstrahlvakuum bis zu einer Badtemperatur von 200°C alle destillierbaren Anteile ab, so erhält man nach der gaschromatographischen Analyse weitere 4 g (entsprechend etwa 4% der Theorie) 2,4,5-Trichlorpyrimidin.

Durch Rektifikation an einer Ag-verspiegelten Füllkörperkolonne von 1 m Länge wird 2,4,5-Trichlorpyrimidin bei $Kp_{12}$ 94—96°C in einer Reinheit von über 99% erhalten.

Das Ausgangsprodukt (2-Cyanethyl)-methyl-dithiocarbamid-säuremethylester wurde folgendermaßen erhalten:

Zu einer auf 5°C abgekühlten Lösung von 203 g (5,07 Mol) Natriumhydroxid in 1800 ml Wasser läßt man unter Außenkühlung mit Eis/Wasser zuerst 420 g (5,0 Mol) 3-Methyl-amino-propionitril und danach während etwa 10 Minuten 400 g (5,26 Mol) Schwefelkohlenstoff fließen. Anschließend wird unter weiterer Kühlung im Eisbad ca. 1,5 Stunden kräftig nachgerührt, wobei eine praktisch homogene Phase entsteht. Unter weiterer Eiskühlung werden nun 650 g (5,16 Mol) Dimethylsulfat in dem Maße zugetropft, daß die Reaktionstemperatur ca. 30°C nicht übersteigt. Danach wird die sich abscheidende ölige Schicht gründlich mit Wasser gewaschen, worauf sie zu einer farblosen kristallinen Masse von reinem (2-Cyanethyl)-methyldithiocarbamidsäuremethylester erstarrt. Nach dem Absaugen und Trocknen Schmelzpunkt 45 bis 46°C. Die Verbindung zeigt ein charakteristisches IR-Spektrum mit folgenden Banden (in $cm^{-1}$): 2250, 1490, 1425, 1380, 1300, 1250, 1195, 1100, 1030, 985, 955, 755.

Beispiel 2

Man verfährt analog Beispiel 1 inclusive der Nachchlorierung bei 150—180°C mit dem Unterschied, daß statt 670 g (6,5 Mol) Schwefeldichlorid nur 360,5 g (3,5 Mol) Schwefeldichlorid vorgelegt werden. Die Destillate enthalten nach gaschromatographischer Analyse 51,6 g (entsprechend 56,3% der Theorie) 2,4,5-Trichlorpyrimidin.

**Patentansprüche**

1. Verfahren zur Herstellung von 2,4,5-Trichlorpyrimidin, dadurch gekennzeichnet, daß man Verbindungen der Formel

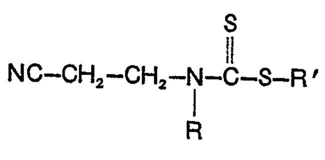

worin R und R′ für $C_1$—$C_4$-Alkyl stehen bei Temperaturen von 0 bis 50°C mit weniger als 7,5 Mol Chlor, insbesondere 3,5 bis 7,0 Mol Chlor, bezogen auf 1 Mol Ausgangsverbindung umsetzt und anschließend in Abwesenheit von Chlor auf Temperaturen von 100— 150°C, insbesondere 110—140°C, nacherhitzt.

2. Verfahren nach Anspruch 1, dadurch gekennzeichnet, daß man bei Temperaturen von etwa 30°C bis etwa 40°C chloriert, bis die exotherme Reaktion beendet ist und anschließend in Abwesenheit von Chlor auf Temperaturen von 110—140°C nacherhitzt.

3. Verfahren nach Anspruch 1, dadurch gekennzeichnet, daß man nach dem Erhitzen auf 100—150°C in Abwesenheit von Chlor mit elementarem Chlor bei 150—200°C nachchloriert.

**Revendications**

1. Procédé de production de 2,4,5-trichloropyrimidine, caractérisé en ce qu'on fait réagir des composé de formule:

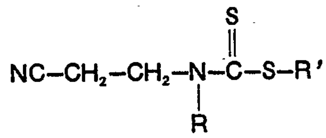

dans laquelle

R et R' représentent un groupe alkyle en $C_1$ à $C_4$, à des températures de 0 à 50°C avec moins de 7,5 moles de chlore, notamment 3,5 à 7,0 moles de chlore, par rapport à une mole de composé de départ, et on chauffe ensuite en l'absence de chlore à des températures de 100 à 150°C, notamment de 110 à 140°C.

2. Procédé suivant la revendication 1, caractérisé en ce qu'on effectue la chloration à des températures d'environ 30°C à environ 40°C jusqu'à ce que la réaction exothermique soit terminée, et on chauffe ensuite à des températures de 110 à 140°C en l'absence de chlore.

3. Procédé suivant la revendication 1, caractérisé en ce que, après le chauffage à 100—150°C en l'absence de chlore, on effectue une chloration subséquente à 150—200°C avec du chlore élémentaire.

**Claims**

1. Process for the preparation of 2,4,5-trichloropyrimidine, characterised in that compounds of the formula

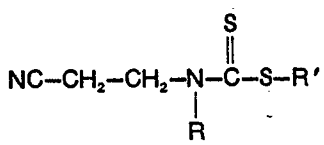

wherein

R and R' represent $C_1$—$C_4$-alkyl are reacted at temperatures from 0 to 50°C with less than 7.5 mols of chlorine, in particular 3.5 to 7.0 mols of chlorine, related to 1 mol of the starting compound, and the reaction products are then after-heated to temperatures of 100—150°C, in particular 110—140°C, in the absence of chlorine.

2. Process according to Claim 1, characterised in that the chlorination is carried out at temperatures from about 30°C to about 40°C until the exothermic reaction has ended, and the reaction products are then after-heated to temperatures of 110—140°C in the absence of chlorine.

3. Process according to Claim 1, characterised in that after heating to 100—150°C in the absence of chlorine, the reaction products are after-chlorinated at 150—200°C with elementary chlorine.